# EUROPEAN PATENT SPECIFICATION

(11) **EP 4 578 526 B1**
(45) Date of publication and mention of the grant of the patent: **06.05.2026**
(21) Application number: 24166468.9
(22) Date of filing: 26.03.2024
(51) Int. Cl.: B01D 3/02, B01D 5/00

(54) **NMP BATCH DISTILLATION DEVICE AND PROCESS**
NMP-CHARGENDESTILLATIONSVORRICHTUNG UND -VERFAHREN
DISPOSITIF ET PROCEDE DE DISTILLATION DISCONTINUE DE NMP

(30) Priority: 29.12.2023 CN 202311863977
(43) Date of publication of application: 02.07.2025
(73) Proprietor: Guangdong Tian Rui De New Energy Technology Co., Ltd., Shenzhen 518000 (CN)
(72) Inventor: YAN, Yongjun, Shenzhen (CN); LI, Tianyou, Shenzhen (CN); MENG, Jili, Shenzhen (CN); ZHU, Chunfang, Shenzhen (CN)
(74) Representative: Chung, Hoi Kan

(56) References cited:
- CN-A- 109 336 803
- CN-B- 102 125 770
- CN-B- 111 494 977
- CN-B- 111 574 423

## Description

### TECHNICAL FIELD

The present invention relates to the field of NMP recovery liquid purification technologies, and in particular, to an NMP batch distillation device and process.

### BACKGROUND

Differences in volatility of components of a liquid mixture are utilized in distillation for vaporizing a part of the liquid mixture and then condensing a part of vapor, so as to implement separation of the components of the liquid mixture. Distillation can be classified into continuous distillation and batch distillation according to different operating manners. Batch distillation means adding a batch of to-be-treated materials to a tower kettle of a distillation tower all at once, then performing heating for distillation until a product from the tower kettle or the top of the tower satisfies a requirement, and discharging nonconforming materials. After the materials are discharged, a new batch of materials is added for distillation. The distillation tower does not distinguish between a rectifying section and a stripping section. During batch distillation operation, liquid in the tower kettle is indirectly heated to boiling, and vapor generated in the tower kettle rises into the distillation tower, where heat and mass exchange are performed. The rising vapor in the tower is led from the top of the tower to a condenser, and a part of condensate liquid obtained from the condenser is then led to a tray at the top of the tower as reflux. If there is a need to obtain distillates with different boiling point ranges, several storage tanks need to be disposed to collect them separately based on different boiling points. Batch distillation is generally performed until composition of the liquid in the tower kettle satisfies specified composition. Compared with continuous distillation, batch distillation has the following features: Materials are fed in batches in batch distillation, and therefore materials cannot be discharged continuously from the tower kettle or the top of the tower. A concentration of discharged materials changes over time. Batch distillation has lower production capacity, but requires a lower construction investment without a need for precise and expensive control instruments, and is suitable for small-scale production.

1-Methyl-2-pyrrolidone (NMP) is an important chemical solvent with low toxicity, high boiling point, a strong dissolving capability, and good selectivity and stability. 1-Methyl-2-pyrrolidone (NMP) is widely used in lithium battery manufacturing. Currently, there are many methods for NMP recovery in the lithium battery industry, and the most widely used method is continuous distillation for a liquid mixture containing NMP, which can effectively remove impurities such as water.

Batch distillation is generally not used for NMP recovery in the industry, and reasons are as follows: NMP is stable in a neutral condition, but is prone to ring opening to form acid and other products in an acid or alkaline condition, and oxygen, water, and temperature have impact on decomposition of NMP. Feed liquid for batch distillation stays for a long time in the tower kettle of the distillation tower, and if the feed liquid for batch distillation is at high temperature for a long time, NMP in NMP waste liquid reacts with dissolved oxygen in raw materials, resulting in a decrease in a product recovery rate and affecting a purified amount of the NMP waste liquid. However, a device that can implement continuous distillation has many components and a large volume, and requires a higher device investment. Installations wherein NMP is purified by distillation are disclosed in CN109336803A and CN111494977B.

### SUMMARY

A main objective of the present invention is to propose an NMP batch distillation device and process, so as to solve problems of a complex structure of an existing NMP recovery device and a small recovered and purified amount of NMP.

To solve the foregoing technical problems, technical solution 1 used in the present invention is as follows: An NMP batch distillation device is provided, including a batch distillation tower, a scraper falling-film evaporation reboiler, a reboiler pump, a tower top condenser, a tower top vacuum condenser, a raw material and intermediate product transfer pump, a water receiving tank, an intermediate product receiving tank, a product receiving tank, and a reflux controller.

The top of the batch distillation tower is connected to the tower top condenser, one outlet of the tower top condenser is connected to the tower top vacuum condenser, and another outlet of the tower top condenser is connected to the reflux controller.

One outlet of the reflux controller is connected to the batch distillation tower, and another outlet of the reflux controller is connected to the water receiving tank, the intermediate product receiving tank, and the product receiving tank.

The raw material and intermediate product transfer pump is connected to the batch distillation tower, the intermediate product receiving tank, and a raw material feed pipe.

The reboiler pump is connected to the batch distillation tower to form a deoxygenation circulation passage, wherein the reboiler pump has a gas inlet configured to introduce nitrogen, by the deoxygenation circulation passage, the reboiler pump pumps out NMP waste liquid from the batch distillation tower, and transfers the NMP waste liquid back to a tower kettle of the batch distillation tower after the nitrogen introduced by the reboiler pump is mixed with the NMP waste liquid and the reboiler pump is also connected to the scraper falling-film evaporation reboiler to form a passage for transferring the NMP waste liquid to the scraper falling-film evaporation reboiler for heating and evaporating, and the scraper falling-film evaporation reboiler is connected to the batch distillation tower, such that a gas phase part of the NMP waste liquid enters a column of the batch distillation tower, and a liquid phase part of the NMP waste liquid that is not vaporized enters the tower kettle of the batch distillation tower, and the reboiler pump is a magnetic gas-liquid mixing pump.

The scraper falling-film evaporation reboiler is connected to the batch distillation tower.

To solve the foregoing technical problems, technical solution 2 used in the present invention is as follows: An NMP batch distillation process is provided, and the process is based on the foregoing NMP batch distillation device.

In an embodiment, the NMP batch distillation process includes a pre-treatment stage and a distillation stage, where the pre-treatment stage includes the following steps:
transferring NMP waste liquid to the batch distillation tower by using the raw material and intermediate product transfer pump;
introducing nitrogen into the batch distillation tower to replace air in the batch distillation tower;
starting a vacuum system of the batch distillation tower to maintain a pressure of 8 to 9 kPa at the top of the tower, and starting a jacketed heating system of a tower kettle, to perform total reflux at the top of the tower;
performing deoxygenation treatment when a temperature of the NMP waste liquid in the tower kettle of the batch distillation tower reaches 0 to 6 °C below a bubble point temperature, where a procedure of the deoxygenation treatment is as follows: the reboiler pump pumps out the NMP waste liquid from the batch distillation tower, and transfers the NMP waste liquid back to the tower kettle of the batch distillation tower after nitrogen is mixed with the NMP waste liquid;
after the deoxygenation treatment is completed, closing the gas inlet of the reboiler pump and a passage between the reboiler pump and the batch distillation tower, and opening a passage between the reboiler pump and the scraper falling-film evaporation reboiler, to heat and evaporate the NMP waste liquid;
during the heating procedure, the scraper falling-film evaporation reboiler evaporates the NMP waste liquid, a gas phase part enters the column of the batch distillation tower, and a liquid phase part that is not vaporized enters the tower kettle of the batch distillation tower; and
when a temperature of the tower kettle of the batch distillation tower reaches a specified temperature and a reflux temperature of the batch distillation tower is stable, performing the distillation stage, where the distillation stage includes the following steps:
   opening a passage between the reflux controller and the water receiving tank, keeping the pressure at the top of the batch distillation tower unchanged, and adjusting a reflux ratio and heating power, to perform water extraction;
   closing the passage between the reflux controller and the water receiving tank, opening a passage between the reflux controller and the intermediate product receiving tank, and adjusting the reflux ratio, the heating power, and the pressure at the top of the batch distillation tower, to perform intermediate product extraction; and
   closing the passage between the reflux controller and the intermediate product receiving tank, opening a passage between the reflux controller and the product receiving tank, keeping the pressure at the top of the batch distillation tower unchanged, and adjusting the reflux ratio and the heating power, to perform NMP product extraction.

The beneficial effects of the present invention are as follows: In the NMP batch distillation device, the gas-liquid mixing pump is used as the reboiler pump, and cooperates with a vacuum environment of the batch distillation tower, to deoxygenate a raw material before batch distillation starts (dissolved oxygen in the liquid phase causes hydrolysis and oxidation of NMP, and therefore removal of the oxygen in the liquid phase is particularly important for reaching a good recovery rate and degree of purity in batch distillation). This helps reduce side reactions, reduce oxidation and decomposition of NMP, and increase the recovered and purified amount of NMP. The scraper falling-film evaporation reboiler is used as the reboiler, and the material stays in the scraper falling-film evaporation reboiler for a short time, so that a probability of a side reaction is reduced, thereby reducing the oxidation and decomposition of NMP and increasing the recovered and purified amount of NMP to a greater extent. The entire NMP batch distillation device has a small quantity of components, an investment cost is low, and an overall structure is simple, which is suitable for small skid-mounted design.

The NMP batch distillation process integrates pre-treatment, dehydration, refining, and heavy component treatment of original continuous distillation into one device. One NMP batch distillation device can perform treatment of different stages on the NMP waste liquid, which helps simplify an NMP purification procedure and increase the recovered and purified amount of NMP. The NMP batch distillation process is simple and practical, and can be well adapted to characteristics of small production businesses in the lithium battery industry, such as a small purified amount of NMP waste liquid and unstable NMP content of the waste liquid.

### BRIEF DESCRIPTION OF DRAWINGS

To describe technical solutions in embodiments of the present invention or in a conventional technology more clearly, the following briefly describes accompanying drawings that are needed for descriptions of embodiments or the conventional technology. It is clear that the accompanying drawings in the following descriptions show merely some embodiments of the present invention, and a person of ordinary skill in the art may still derive other drawings from structures shown in these accompanying drawings without creative efforts.

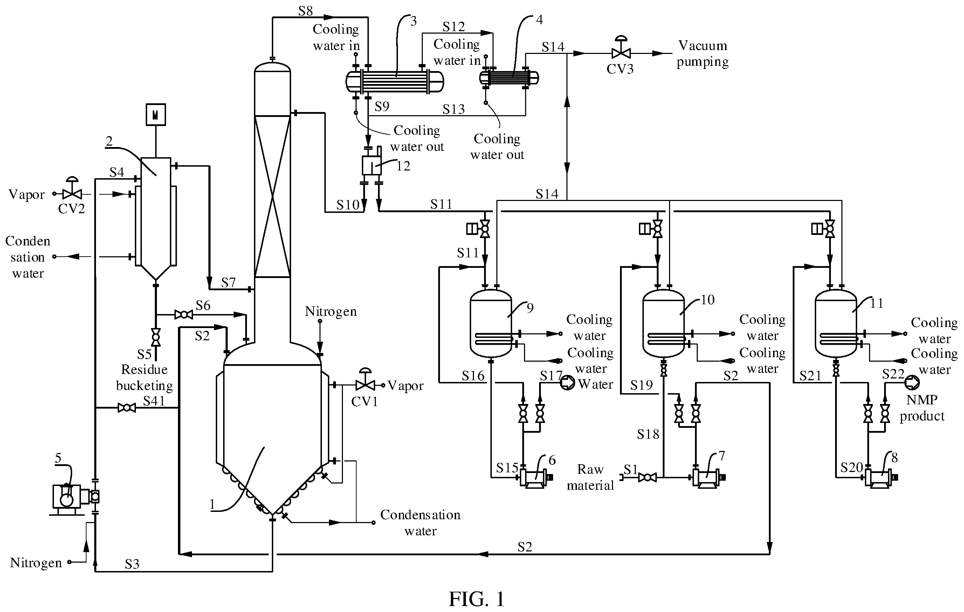

FIG. 1 is a simplified schematic diagram of a structure of an NMP batch distillation device according to Embodiment 1 of the present invention.

Reference numerals:
1: batch distillation tower; 2: scraper falling-film evaporation reboiler; 3: tower top condenser; 4: tower top vacuum condenser; 5: reboiler pump; 6: water transfer pump; 7: raw material and intermediate product transfer pump; 8: product transfer pump; 9: water receiving tank; 10: intermediate product receiving tank; 11: product receiving tank; 12: reflux controller;
S1: first pipe; S2: second pipe; S3: third pipe; S4: fourth pipe; S41: branch pipe; S5: fifth pipe; S6: sixth pipe; S7: seventh pipe; S8: eighth pipe; S9: ninth pipe; S10: tenth pipe; S11: eleventh pipe; S12: twelfth pipe; S13: thirteenth pipe; S14: fourteenth pipe; S15: fifteenth pipe; S16: sixteenth pipe; S17: seventeenth pipe; S18: eighteenth pipe; S19: nineteenth pipe; S20: twentieth pipe; S21: twenty-first pipe; S22: twenty-second pipe.

### DESCRIPTION OF EMBODIMENTS

Achievement of objectives, functional features, and advantages of the present invention are further described with reference to embodiments and accompanying drawings.

The following clearly and completely describes technical solutions in embodiments of the present invention with reference to the accompanying drawings in embodiments of the present invention.

It should be noted that, if embodiments of the present invention involve directional indications such as above, below, left, right, front, and back, the directional indications are merely used to explain relative position relationships, movement conditions, or the like of components in a specific attitude, as shown in the accompanying drawings. If the specific attitude changes, the directional indications change accordingly.

In addition, if there are descriptions involving "first", "second", and the like in embodiments of the present invention, the descriptions of "first", "second", and the like are merely intended for an objective of description, and shall not be understood as an indication or implication of relative importance or implicit indication of a quantity of indicated technical features. Therefore, a feature defined by "first" or "second" may explicitly or implicitly include at least one feature.

In addition, "and/or" in this specification means that three parallel solutions are included. For example, "A and/or B" indicates the following three solutions: Only A exists, only B exists, or both A and B exist. In addition, the technical solutions in various embodiments can be combined with each other, which should be based on implementation by a person of ordinary skill in the art. When the combination of technical solutions is contradictory or cannot be implemented, it should be considered that such a combination of technical solutions does not exist, and is not within the protection scope claimed by the present invention.

In this application, unless otherwise specified and limited, terms such as "mount", "link", "connect", and "fasten" should be understood broadly. For example, the term "connect" may indicate a fixed connection, a detachable connection, or integration; may indicate a mechanical connection or an electrical connection; may indicate a direct connection or an indirect connection implemented by using an intermediate medium; or may indicate communication inside two elements or an interaction relationship between two elements. A person of ordinary skill in the art may interpret specific meanings of the foregoing terms in this application according to specific cases.

### Embodiment 1

Refer to FIG. 1. Embodiment 1 of the present invention is as follows: An NMP batch distillation device is provided, including a batch distillation tower 1, a scraper falling-film evaporation reboiler 2, a reboiler pump 5, a tower top condenser 3, a tower top vacuum condenser 4, a water transfer pump 6, a raw material and intermediate product transfer pump 7, a product transfer pump 8, a water receiving tank 9, an intermediate product receiving tank 10, a product receiving tank 11, and a reflux controller 12. The scraper falling-film evaporation reboiler 2 can treat an NMP scrap material containing tiny solid particulate matter. Compared with a kettle reboiler and a thermosiphon reboiler commonly used in a conventional technology, the scraper falling-film evaporation reboiler 2 is not blocked even after being used for a long time, which helps the NMP batch distillation device operate stably for a long time.

The top of the batch distillation tower 1 is connected to the tower top condenser 3 through an eighth pipe S8. One outlet of the tower top condenser 3 is connected to the tower top vacuum condenser 4 through a twelfth pipe S12, and another outlet is connected to the reflux controller 12 through a ninth pipe S9. The tower top vacuum condenser 4 is connected to the reflux controller 12 through a thirteenth pipe S13, and is connected, through a fourteenth pipe S14, to the outside, the water receiving tank 9, the intermediate product receiving tank 10, or the product receiving tank 11.

One outlet of the reflux controller 12 is connected to the batch distillation tower 1 through a tenth pipe S10, and another outlet is connected, through an eleventh pipe S11, to the water receiving tank 9, the intermediate product receiving tank 10, and the product receiving tank 11.

The raw material and intermediate product transfer pump 7 is connected to the batch distillation tower 1, the intermediate product receiving tank 10, and a raw material feed pipe (that is, a first pipe S1). NMP waste liquid is introduced into the first pipe S1 to enter the NMP batch distillation device. The raw material and intermediate product transfer pump 7 is connected to the batch distillation tower 1 through a second pipe S2, and is connected to the intermediate product receiving tank 10 through a nineteenth pipe S19. The intermediate product receiving tank 10 is connected to the first pipe S1 through an eighteenth pipe S18.

The reboiler pump 5 is connected to the batch distillation tower through a third pipe S3, and is connected to the scraper falling-film evaporation reboiler 2 through a fourth pipe S4. The reboiler pump 5 is a gas-liquid mixing pump, and the reboiler pump 5 has a gas inlet configured to introduce nitrogen. It is easy to understand that the gas inlet is not merely used for entering of nitrogen, and may be used for entering of other inert gases, such as argon and helium. Using argon or helium instead of nitrogen to perform resolving-based deoxygenation is a direct replacement of a common means in the art. In addition, the gas-liquid mixing pump may be a vortex pump, a centrifugal spiral microbubble pump, or the like. The pump is a magnetic type pump. The fourth pipe S4 is connected to a branch pipe S41, and the reboiler pump 5 is directly connected to the batch distillation tower 1 through the branch pipe S41. It is easy to understand that the gas-liquid mixing pump can implement nitrogen filling for deoxygenation, mix materials, and improve heat transfer.

The top of the scraper falling-film evaporation reboiler 2 is connected to the batch distillation tower 1 through a seventh pipe S7, and the bottom of the scraper falling-film evaporation reboiler 2 is connected to the batch distillation tower 1 through a sixth pipe S6. The bottom of the scraper falling-film evaporation reboiler 2 is further connected to a fifth pipe S5, and the fifth pipe S5 is configured to discharge a residue.

The bottom of the water receiving tank 9 is connected to the water transfer pump 6 through a fifteenth pipe S15. The water transfer pump 6 is connected to the top of the water receiving tank 9 through a sixteenth pipe S16, and discharges water outward through a seventeenth pipe S17.

The bottom of the product receiving tank 11 is connected to the product transfer pump 8 through a twentieth pipe S20. The product transfer pump 8 is connected to the top of the product receiving tank 11 through a twenty-first pipe S21, and outputs an NMP product outward through a twenty-second pipe S22.

The batch distillation tower 1 is preferably filled with a BX500 mesh filler or a mesh filler with equivalent performance. A height of the filler is 3 to 6 m, and there is a total of one layer of filler. Compared with a current continuous distillation tower in which there are multi-layer fillers and a height of the fillers in the single tower is 6 to 10 m, the batch distillation tower 1 in the NMP batch distillation device has a smaller volume, which is more conducive to miniaturization of the NMP batch distillation device.

This embodiment further provides an NMP batch distillation process, and the process is based on the foregoing NMP batch distillation device.

In an embodiment, the process includes a pre-treatment stage and a distillation stage, where the pre-treatment stage includes the following steps:
transferring NMP waste liquid (a raw material) to the batch distillation tower by using the raw material and intermediate product transfer pump;
introducing nitrogen into the batch distillation tower to replace air in the batch distillation tower;
starting a vacuum system of the batch distillation tower to maintain a pressure of 8 to 9 kPa at the top of the tower, and starting a jacketed heating system of a tower kettle, to perform total reflux at the top of the tower;
performing deoxygenation treatment when a temperature of the NMP waste liquid in the tower kettle of the batch distillation tower reaches 0 to 6 °C below a bubble point temperature, where a procedure of the deoxygenation treatment is as follows: the reboiler pump pumps out the NMP waste liquid from the batch distillation tower, and transfers the NMP waste liquid back to the tower kettle of the batch distillation tower after nitrogen is mixed with the NMP waste liquid; and preferably, the procedure of the deoxygenation treatment is repeated at least twice;
after the deoxygenation treatment is completed, closing the gas inlet of the reboiler pump and a passage between the reboiler pump and the batch distillation tower, and opening a passage between the reboiler pump and the scraper falling-film evaporation reboiler, to heat and evaporate the NMP waste liquid;
during the heating procedure, the scraper falling-film evaporation reboiler evaporates the NMP waste liquid, a gas phase part enters the column of the batch distillation tower, and a liquid phase part that is not vaporized enters the tower kettle of the batch distillation tower; and
when a temperature of the tower kettle of the batch distillation tower reaches a specified temperature and a reflux temperature of the batch distillation tower is stable, performing the distillation stage, where the distillation stage includes the following steps:
   opening a passage between the reflux controller and the water receiving tank, keeping the pressure at the top of the batch distillation tower unchanged, and adjusting a reflux ratio and heating power, to perform water extraction; and in this case, the heating power of the jacketed heating system is at full load, the pressure at the top of the batch distillation tower is 8 to 9 kPa, and the reflux ratio at the top of the batch distillation tower is adjusted to 0.1 to 0.3;
   closing the passage between the reflux controller and the water receiving tank, opening a passage between the reflux controller and the intermediate product receiving tank, and adjusting the reflux ratio, the heating power, and the pressure at the top of the batch distillation tower, to perform intermediate product extraction; and in this case, the heating power of the jacketed heating system is 30% to 40% of the full load, the pressure at the top of the batch distillation tower is reduced from 8 to 9 kPa to 2 to 3 kPa, and the reflux ratio at the top of the batch distillation tower is adjusted to 0.2 to 0.4; and
   closing the passage between the reflux controller and the intermediate product receiving tank, opening a passage between the reflux controller and the product receiving tank, keeping the pressure at the top of the batch distillation tower unchanged, and adjusting the reflux ratio and the heating power, to perform NMP product extraction; and in this case, the heating power of the jacketed heating system is 30% to 40% of the full load, the pressure at the top of the batch distillation tower remains unchanged at 2 to 3 kPa, and the reflux ratio at the top of the batch distillation tower is adjusted to 0.5 to 1.2.

When the NMP batch distillation process is actually implemented, the intermediate product extracted in the distillation stage may be introduced into the raw material feed pipe to be used as a part of a raw material for next implementation of the NMP batch distillation process.

Compared with an existing continuous production process in which a temperature of a tower kettle of a distillation tower remains at 120 °C approximately, a controlled temperature of the tower kettle of the batch distillation tower is low, which is approximately 104 °C. Therefore, a probability of a side reaction is low, which helps increase a purified amount of the NMP product.

This embodiment further provides an implementation case to allow readers to more fully understand the technical solution. In this case, each batch of NMP waste liquid is 700 kg, and raw material components are as follows: NMP content: 560 kg; water content: 138.32 kg; and heavy component content: 1.68 kg. A diameter of the batch distillation tower is 350 mm, a filler layer is 3500 mm, and a wire mesh filler is used.

The NMP waste liquid of 700 kg is added to the batch distillation tower through the raw material and intermediate product transfer pump, and then an interface of a nitrogen pipe of the batch distillation tower is opened, to replace air in the batch distillation tower. The vacuum system of the batch distillation tower is started to control pressure at the top of the batch distillation tower to be 8 kPa, and the jacketed heating system of the tower kettle of the batch distillation tower is started. Heating power is approximately 150 kW, and total reflux is used at the top of the tower. Before a formal distillation operation is performed, the NMP waste liquid needs to be deoxygenated. After deoxygenation of the NMP waste liquid, dissolved oxygen in the NMP waste liquid can be reduced, so that side reactions can be reduced at a high temperature. When a temperature of the tower kettle of the batch distillation tower reaches 51 °C, the temperature of the tower kettle of the batch distillation tower remains unchanged. The reboiler pump is a magnetic gas-liquid mixing pump (vortex pump), and a small quantity of nitrogen is introduced into an inlet of the pump. The nitrogen is fully dissolved in the NMP waste liquid due to pressurized mixing of the nitrogen in the pump. Therefore, the nitrogen can be mixed with the NMP waste liquid without a need for a stirrer. Then in the tower kettle of the batch distillation tower, dissolved gases (including oxygen) in the NMP waste liquid close to a bubble point are removed, because the pressure in the tower kettle is reduced and the NMP waste liquid circulates continuously and flows up and down under an action of the reboiler pump. After introduction of the nitrogen is completed, the introduction of the nitrogen is stopped, and the NMP waste liquid is stabilized for a specific period of time under a cyclic operating condition of the reboiler pump, which allows the dissolved oxygen in the NMP waste liquid to be fully removed. Then the foregoing procedure is repeated twice, and it can be considered that removal of the dissolved oxygen in the NMP waste liquid is completed. A principle of this deoxygenation procedure can be considered as vacuum deoxygenation and resolving-based deoxygenation. After the deoxygenation is completed, the temperature of the tower kettle of the batch distillation tower is controlled to continue to increase until the temperature of the tower kettle is 56.2 °C, and stable reflux is formed at the top of the batch distillation tower. This stage takes approximately 35 minutes.

The passage between the reflux controller and the water receiving tank is opened, to prepare to start water extraction. A reflux ratio at the top of the batch distillation tower remains unchanged at 0.2. As water is gradually extracted, the temperature of the tower kettle gradually increases. As an increasing quantity of water is extracted, an NMP concentration in a gas phase is increasingly high. Because latent heat of vaporization of NMP is lower than latent heat of vaporization of water, a flow quantity of the gas phase in the batch distillation tower is also increasingly high. Excessively high heating load causes an increasingly high flow quantity of the gas phase in the batch distillation tower. When the temperature of the tower kettle increases to 85 °C, the reflux ratio is adjusted to 0.1, and the heating power is reduced from 150 kW to 110 kW within 6 minutes until the temperature of the tower kettle increases to 100 °C. This stage takes approximately 45 minutes.

The passage between the reflux controller and the water receiving tank is closed, and the passage between the reflux controller and the intermediate product receiving tank is opened, to prepare to start intermediate product extraction. The heating load is adjusted to 40 kW, and the reflux ratio is adjusted to 0.3. Pressure of the tower top condenser is reduced through the adjustment, to control the temperature of the tower kettle to remain at 104 °C until the pressure of the tower top condenser is reduced to 3 kPa. Subsequently, the temperature of the tower kettle is controlled to remain at 104 °C by controlling the reflux ratio, and water content of a material in the tower top condenser is less than 0.001. This stage takes approximately 40 minutes. Water content of a material in the intermediate product receiving tank is approximately 40%, and the material may be transferred back to the tower kettle of the batch distillation tower during next batch of distillation operation and purified again.

The passage between the reflux controller and the intermediate product receiving tank is closed, and the passage between the reflux controller and the product receiving tank is opened, to prepare to start NMP product extraction. The heating load is adjusted to 40 kW within 3 minutes, and the reflux ratio is controlled to be 0.7. The extraction continues until a liquid-holding capacity of the tower kettle of the batch distillation tower is approximately 10 kg, and the single distillation operation is completed. Residues of the tower kettle are packed into barrels and sent out for treatment. This stage takes approximately 3 hours and 5 minutes.

After this batch is completed, composition of residual liquid and filler liquid-holding materials in receiving tanks and the tower kettle is shown in Table 1.

**Table 1 Content and composition of each component after batch distillation**

| | Water receiving tank | Intermediat e product receiving tank | Product receiving tank | Residual liquid in tower kettle | Liquid-holdi ng capacity of tray | Total |
|---|---|---|---|---|---|---|
| NMP/kg | 4.19816×10⁻¹¹ | 22.0709 | 513.586 | 8.67471 | 15.66839 | 560 |
| Water/kg | 122.745 | 15.5717 | 0.00304592 | 0 | 0 | 138.32 |
| Heavy component/ kg | 0 | 1.3373×10⁻⁶ | 0.0189234 | 1.32456 | 0.33606 | 1.68 |
| Total/kg | 122.745 | 37.6426 | 513.608 | 9.99927 | 16.00445 | 700 |

The following describes characteristics of the technical solution.
1. A characteristic of batch distillation is that the material stays for a long time. If the material is at high temperature for a long time, NMP in the NMP waste liquid reacts with the dissolved oxygen in the raw material, resulting in a decrease in a product recovery rate. There is a need to deoxygenate the NMP waste liquid before batch distillation starts, to reduce side reactions. This technical solution uses a magnetic gas-liquid mixing pump (vortex pump). The pump has characteristics of a small flow quantity and high lift, and has a leak-free advantage of a magnetic pump. A suction opening of the gas-liquid mixing pump sucks nitrogen, and a high-speed rotating pump impeller mixes and stirs the NMP waste liquid and the nitrogen. The nitrogen is fully dissolved in the waste liquid due to the pressurized mixing in the pump, and therefore mixed waste liquid can be obtained without a need for a stirrer. Then in the tower kettle of the distillation tower, dissolved gases in the NMP waste liquid close to a bubble point are removed because the material in the tower kettle circulates continuously and flows up and down under an action of the pump, so that side reactions can be reduced at a high temperature. In addition, the magnetic gas-liquid mixing pump (vortex pump) can be used as a feed pump of the scraper falling-film evaporation reboiler and a mixing pump of the tower kettle of the batch distillation tower. The mixing of the materials in the tower kettle optimizes heat transfer, and there is no need to dispose a stirrer for the tower kettle. In general, deoxygenation of the NMP waste liquid reduces oxidation and decomposition of NMP, and a product purification amount can increase by 3 to 5%.
2. Because the NMP waste liquid contains a trace quantity of solid matter, the scraper falling-film evaporation reboiler is used, to prevent the trace quantity of solid matter from accumulating on device components such as the reboiler, where maximum solid content of a liquid phase can reach 15%. Other forms of reboilers such as a kettle reboiler may be blocked during treatment of residues of the tower kettle. In addition, the scraper falling-film evaporator has high heat transfer efficiency, and a total heat transfer coefficient is 1750 to 7000 W/(m²·°C). The material stays in the scraper falling-film evaporation reboiler for a short time, and a probability of a side reaction is low.
3. The batch distillation tower uses a BX500 wire mesh filler. The BX500 wire mesh filler has low unit theoretical plate pressure and a small liquid-holding capacity and is easily wet, and the effect is ideal.
4. Batch distillation integrates pre-treatment, dehydration, refining, and heavy component treatment of original continuous distillation. Stages of batch distillation are divided into a total reflux and heating stage, a dehydration stage, and a product refining stage. Heating power is different when one device implements different treatment stages. The heating power at the dehydration stage is large, while the heating power at the refining stage is small.
5. A controlled temperature of the tower kettle of the batch distillation tower is low, and a probability of a side reaction is low.

The foregoing embodiments are merely optional embodiments of the present invention. The scope of the invention is limited by the appended claims.

## Claims

1. A 1-Methyl-2-pyrrolidone batch distillation device, comprising a batch distillation tower (1), a scraper falling-film evaporation reboiler (2), a reboiler pump (5), a tower top condenser (3), a tower top vacuum condenser (4), a raw material and intermediate product transfer pump (7), a water receiving tank (9), an intermediate product receiving tank (10), a product receiving tank (11), and a reflux controller (12), wherein
the top of the batch distillation tower (1) is connected to the tower top condenser (3), one outlet of the tower top condenser (3) is connected to the tower top vacuum condenser (4), and another outlet of the tower top condenser (3) is connected to the reflux controller (12);
one outlet of the reflux controller (12) is connected to the batch distillation tower (1), and another outlet of the reflux controller (12) is connected to the water receiving tank (9), the intermediate product receiving tank (10), and the product receiving tank (11);
the raw material and intermediate product transfer pump (7) is connected to the batch distillation tower (1), the intermediate product receiving tank (10), and a raw material feed pipe (S1);
the reboiler pump (5) is connected to the batch distillation tower (1) to form a deoxygenation circulation passage, wherein the reboiler pump (5) has a gas inlet configured to introduce nitrogen, by the deoxygenation circulation passage, the reboiler pump pumps out 1-Methyl-2-pyrrolidone waste liquid from the batch distillation tower, and transfers the 1-Methyl-2-pyrrolidone waste liquid back to a tower kettle of the batch distillation tower after the nitrogen introduced by the reboiler pump (5) is mixed with the 1-Methyl-2-pyrrolidone waste liquid; and
the reboiler pump (5) is also connected to the scraper falling-film evaporation reboiler (2) to form a passage for transferring the 1-Methyl-2-pyrrolidone waste liquid to the scraper falling-film evaporation reboiler (2) for heating and evaporating, and the scraper falling-film evaporation reboiler (2) is connected to the batch distillation tower (1), such that a gas phase part of the 1-Methyl-2-pyrrolidone waste liquid enters a column of the batch distillation tower (1), and a liquid phase part of the 1-Methyl-2-pyrrolidone waste liquid that is not vaporized enters the tower kettle of the batch distillation tower, and the reboiler pump (5) is a magnetic gas-liquid mixing pump.

2. The 1-Methyl-2-pyrrolidone batch distillation device according to claim 1, further comprising a water transfer pump (6) connected to the water receiving tank (9).

3. The 1-Methyl-2-pyrrolidone batch distillation device according to claim 1, further comprising a product transfer pump (8) connected to the product receiving tank (11).

4. The 1-Methyl-2-pyrrolidone batch distillation device according to claim 1, wherein the batch distillation tower (1) is filled with a BX500 mesh filler, and a height of the filler is 3 to 6 m.

5. A 1-Methyl-2-pyrrolidone batch distillation process based on the NMP batch distillation device according to any one of claims 1 to 4, comprising a pre-treatment stage and a distillation stage, wherein the pre-treatment stage comprises the following steps:
transferring 1-Methyl-2-pyrrolidone waste liquid to the batch distillation tower (1) by using the raw material and intermediate product transfer pump (7);
introducing nitrogen into the batch distillation tower (1) to replace air in the batch distillation tower (1);
starting a vacuum system of the batch distillation tower (1) to maintain a pressure of 8 to 9 kPa at the top of the tower, and starting a jacketed heating system of a tower kettle, to perform total reflux at the top of the tower;
performing deoxygenation treatment when a temperature of the 1-Methyl-2-pyrrolidone waste liquid in the tower kettle of the batch distillation tower (1) reaches 0 to 6 °C below a bubble point temperature, wherein a procedure of the deoxygenation treatment is as follows: the reboiler pump (5) pumps out the 1-Methyl-2-pyrrolidone waste liquid from the batch distillation tower, and transfers the 1-Methyl-2-pyrrolidone waste liquid back to the tower kettle of the batch distillation tower (1) after nitrogen is mixed with the 1-Methyl-2-pyrrolidone waste liquid;
after the deoxygenation treatment is completed, closing the gas inlet of the reboiler pump (5) and a passage between the reboiler pump (5) and the batch distillation tower (1), and opening a passage between the reboiler pump (5) and the scraper falling-film evaporation reboiler (2), to heat and evaporate the 1-Methyl-2-pyrrolidone waste liquid;
during the heating procedure, the scraper falling-film evaporation reboiler (2) evaporates the 1-Methyl-2-pyrrolidone waste liquid, a gas phase part enters the column of the batch distillation tower (1), and a liquid phase part that is not vaporized enters the tower kettle of the batch distillation tower (1); and
when a temperature of the tower kettle of the batch distillation tower (1) reaches a specified temperature and a reflux temperature of the batch distillation tower (1) is stable, performing the distillation stage, wherein the distillation stage comprises the following steps:
opening a passage between the reflux controller (12) and the water receiving tank (9), keeping the pressure at the top of the batch distillation tower (1) unchanged, and adjusting a reflux ratio and heating power, to perform water extraction;
closing the passage between the reflux controller (12) and the water receiving tank (9), opening a passage between the reflux controller (12) and the intermediate product receiving tank (10), and adjusting the reflux ratio, the heating power, and the pressure at the top of the batch distillation tower (1), to perform intermediate product extraction; and
closing the passage between the reflux controller (12) and the intermediate product receiving tank (10), opening a passage between the reflux controller (12) and the product receiving tank (11), keeping the pressure at the top of the batch distillation tower (1) unchanged, and adjusting the reflux ratio and the heating power, to perform 1-Methyl-2-pyrrolidone product extraction.

6. The 1-Methyl-2-pyrrolidone batch distillation process according to claim 5, wherein the procedure of the deoxygenation treatment is repeated at least twice in the pre-treatment stage.

7. The 1-Methyl-2-pyrrolidone batch distillation process according to claim 5, wherein when the water extraction is performed, the heating power of the jacketed heating system is at full load, the pressure at the top of the batch distillation tower (1) is 8 to 9 kPa, and the reflux ratio at the top of the batch distillation tower (1) is adjusted to 0.1 to 0.3.

8. The1-Methyl-2-pyrrolidone batch distillation process according to claim 5, wherein when the intermediate product extraction is performed, the heating power of the jacketed heating system is 30% to 40% of full load, the pressure at the top of the batch distillation tower (1) is reduced from 8 to 9 kPa to 2 to 3 kPa, and the reflux ratio at the top of the batch distillation tower (1) is adjusted to 0.2 to 0.4.

9. The1-Methyl-2-pyrrolidone batch distillation process according to claim 5, wherein when the 1-Methyl-2-pyrrolidone product extraction is performed, the heating power of the jacketed heating system is 30% to 40% of full load, the pressure at the top of the batch distillation tower (1) remains unchanged at 2 to 3 kPa, and the reflux ratio at the top of the batch distillation tower (1) is adjusted to 0.5 to 1.2.

## Patentansprüche

1. Chargendestillationsvorrichtung für 1-Methyl-2-pyrrolidon, umfassend einen Chargendestillationsturm (1), einen Schaberfallfilmverdampfer-Rückkocher (2), eine Rückkocherpumpe (5), einen Turmkopfkondensator (3), einen Vakuumturmkopfkondensator (4), eine Förderpumpe für Rohmaterial und Zwischenprodukt (7), einen Wasseraufnahmetank (9), einen Zwischenproduktaufnahmetank (10), einen Produktaufnahmetank (11) und einen Rückflussregler (12), wobei
der Kopf des Chargendestillationsturms (1) mit dem Turmkopfkondensator (3) verbunden ist, wobei ein Auslass des Turmkopfkondensators (3) mit dem Vakuumturmkopfkondensator (4) verbunden ist und ein weiterer Auslass des Turmkopfkondensators (3) mit dem Rückflussregler (12) verbunden ist,
wobei ein Auslass des Rückflussreglers (12) mit dem Chargendestillationsturm (1) verbunden ist und ein weiterer Auslass des Rückflussreglers (12) mit dem Wasseraufnahmetank (9), dem Zwischenproduktaufnahmetank (10) und dem Produktaufnahmetank (11) verbunden ist,
wobei die Förderpumpe für Rohmaterial und Zwischenprodukt (7) mit dem Chargendestillationsturm (1), dem Zwischenproduktaufnahmetank (10) und einer Rohstoffzufuhrleitung (S1) verbunden ist,
wobei die Rückkocherpumpe (5) mit dem Chargendestillationsturm (1) verbunden ist, um einen Entsauerstoffungszirkulationskanal zu bilden, wobei die Rückkocherpumpe (5) einen Gaseinlass aufweist, der zum Einleiten von Stickstoff eingerichtet ist, wobei über den Entsauerstoffungszirkulationskanal die Rückkocherpumpe 1-Methyl-2-pyrrolidon-Abfallflüssigkeit aus dem Chargendestillationsturm abpumpt und die 1-Methyl-2-pyrrolidon-Abfallflüssigkeit zurück in einen Turmkessel des Chargendestillationsturms überführt, nachdem der von der Rückkocherpumpe (5) eingeleitete Stickstoff mit der 1-Methyl-2-pyrrolidon-Abfallflüssigkeit vermischt wurde; und
wobei die Rückkocherpumpe (5) weiterhin mit dem Schaberfallfilmverdampfer-Rückkocher (2) verbunden ist, um einen Kanal zum Überführen der 1-Methyl-2-pyrrolidon-Abfallflüssigkeit zum Schaberfallfilmverdampfer-Rückkocher (2) zum Erhitzen und Verdampfen zu bilden, wobei der Schaberfallfilmverdampfer-Rückkocher (2) mit dem Chargendestillationsturm (1) verbunden ist, so dass ein Gasphasenanteil der 1-Methyl-2-pyrrolidon-Abfallflüssigkeit in eine Kolonne des Chargendestillationsturms (1) eintritt, wobei ein nicht verdampfter Flüssigphasenanteil der 1-Methyl-2-pyrrolidon-Abfallflüssigkeit in den Turmkessel des Chargendestillationsturms eintritt, und wobei die Rückkocherpumpe (5) eine magnetische Gas-Flüssigkeits-Mischpumpe ist.

2. Chargendestillationsvorrichtung für 1-Methyl-2-pyrrolidon nach Anspruch 1, die ferner eine mit dem Wasseraufnahmetank (9) verbundene Wasserförderpumpe (6) umfasst.

3. Chargendestillationsvorrichtung für 1-Methyl-2-pyrrolidon nach Anspruch 1, die ferner eine mit dem Produktaufnahmetank (11) verbundene Produktförderpumpe (8) umfasst.

4. Chargendestillationsvorrichtung für 1-Methyl-2-pyrrolidon nach Anspruch 1, wobei der Chargendestillationsturm (1) mit einem Füllkörper mit einer Maschenweite BX500 gefüllt ist und eine Höhe des Füllkörpers 3 bis 6 m beträgt.

5. Chargendestillationsverfahren für 1-Methyl-2-pyrrolidon auf der Grundlage der NMP-Chargendestillationsvorrichtung nach einem der Ansprüche 1 bis 4, umfassend eine Vorbehandlungsstufe und eine Destillationsstufe, wobei die Vorbehandlungsstufe folgende Schritte umfasst:
Überführen der 1-Methyl-2-pyrrolidon-Abfallflüssigkeit in den Chargendestillationsturm (1) unter Verwendung der Förderpumpe für Rohmaterial und Zwischenprodukt (7);
Einleiten von Stickstoff in den Chargendestillationsturm (1), um Luft im Chargendestillationsturm (1) zu ersetzen;
Starten eines Vakuumsystems des Chargendestillationsturms (1), um einen Druck von 8 bis 9 kPa am Turmkopf aufrechtzuerhalten, und Starten eines ummantelten Heizsystems eines Turmkessels, um am Turmkopf einen vollständigen Rückfluss durchzuführen;
Durchführen einer Entsauerstoffungsbehandlung, wenn eine Temperatur der 1-Methyl-2-pyrrolidon-Abfallflüssigkeit im Turmkessel des Chargendestillationsturms (1) 0 bis 6 °C unterhalb einer Blasenpunkt-Temperatur liegt, wobei ein Vorgang der Entsauerstoffungsbehandlung wie folgt ist: die Rückkocherpumpe (5) pumpt die 1-Methyl-2-pyrrolidon-Abfallflüssigkeit aus dem Chargendestillationsturm ab und überführt die 1-Methyl-2-pyrrolidon-Abfallflüssigkeit zurück in den Turmkessel des Chargendestillationsturms (1), nachdem Stickstoff mit der 1-Methyl-2-pyrrolidon-Abfallflüssigkeit vermischt wurde;
Nach Abschluss der Entsauerstoffungsbehandlung, Schließen des Gaseinlasses der Rückkocherpumpe (5) und eines Kanals zwischen der Rückkocherpumpe (5) und dem Chargendestillationsturm (1) und Öffnen eines Kanals zwischen der Rückkocherpumpe (5) und dem Schaberfallfilmverdampfer-Rückkocher (2), um die 1-Methyl-2-pyrrolidon-Abfallflüssigkeit zu erhitzen und zu verdampfen,
wobei während des Erhitzungsvorgangs der Schaberfallfilmverdampfer-Rückkocher (2) die 1-Methyl-2-pyrrolidon-Abfallflüssigkeit verdampft, wobei ein Gasphasenanteil in die Kolonne des Chargendestillationsturms (1) eintritt und ein nicht verdampfter Flüssigphasenanteil in den Turmkessel des Chargendestillationsturms (1) eintritt; und
wenn eine Temperatur des Turmkessels des Chargendestillationsturms (1) eine spezifische Temperatur erreicht und eine Rücklauftemperatur des Chargendestillationsturms (1) stabil ist, Durchführen der Destillationsstufe, wobei die Destillationsstufe die folgenden Schritte umfasst:
Öffnen eines Kanals zwischen dem Rückflussregler (12) und dem Wasseraufnahmetank (9), Beibehalten des Drucks am Kopf des Chargendestillationsturms (1) und Einstellen eines Rückflussverhältnisses und einer Heizleistung, um eine Wasserextraktion durchzuführen;
Schließen des Kanals zwischen dem Rückflussregler (12) und dem Wasseraufnahmetank (9), Öffnen eines Kanals zwischen dem Rückflussregler (12) und dem Zwischenproduktaufnahmetank (10) sowie Einstellen des Rückflussverhältnisses, der Heizleistung und des Drucks am Kopf des Chargendestillationsturms (1), um die Zwischenproduktextraktion durchzuführen; und
Schließen des Kanals zwischen dem Rückflussregler (12) und dem Zwischenproduktaufnahmetank (10), Öffnen eines Kanals zwischen dem Rückflussregler (12) und dem Produktaufnahmetank (11), Beibehalten des Drucks am Kopf des Chargendestillationsturms (1) und Einstellen des Rückflussverhältnisses und der Heizleistung, um die 1-Methyl-2-pyrrolidon-Produktextraktion durchzuführen.

6. Chargendestillationsverfahren für 1-Methyl-2-pyrrolidon nach Anspruch 5, wobei der Vorgang der Entsauerstoffungsbehandlung in der Vorbehandlungsstufe mindestens zweimal wiederholt wird.

7. Chargendestillationsverfahren für 1-Methyl-2-pyrrolidon nach Anspruch 5, wobei bei dem Durchführen der Wasserextraktion die Heizleistung des ummantelten Heizsystems unter Volllast steht, wobei der Druck am Kopf des Chargendestillationsturms (1) 8 bis 9 kPa beträgt und das Rückflussverhältnis am Kopf des Chargendestillationsturms (1) auf 0,1 bis 0,3 eingestellt ist.

8. Chargendestillationsverfahren für 1-Methyl-2-pyrrolidon nach Anspruch 5, wobei bei dem Durchführen der Zwischenproduktextraktion die Heizleistung des ummantelten Heizsystems unter 30 % bis 40 % der Volllast steht, wobei der Druck am Kopf des Chargendestillationsturms (1) von 8 bis 9 kPa auf 2 bis 3 kPa reduziert wird und das Rückflussverhältnis am Kopf des Chargendestillationsturms (1) auf 0,2 bis 0,4 eingestellt ist.

9. Chargendestillationsverfahren für 1-Methyl-2-pyrrolidon nach Anspruch 5, wobei bei dem Durchführen der 1-Methyl-2-pyrrolidon-Produktextraktion die Heizleistung des ummantelten Heizsystems unter 30 % bis 40 % der Volllast steht, wobei der Druck am Kopf des Chargendestillationsturms (1) unverändert bei 2 bis 3 kPa bleibt und das Rückflussverhältnis am Kopf des Chargendestillationsturms (1) auf 0,5 bis 1,2 eingestellt ist.

## Revendications

1. Dispositif de distillation discontinue de 1-méthyl-2-pyrrolidone, comprenant une tour de distillation discontinue (1), un rebouilleur d'évaporation à flux tombant de grattoir (2), une pompe de rebouilleur (5), un condenseur en haut de la tour (3), un condenseur sous vide en haut de la tour (4), une pompe de transfert de matière première et de produit intermédiaire (7), un réservoir de réception d'eau (9), un réservoir de réception de produit intermédiaire (10), un réservoir de réception de produit (11) et un dispositif de commande de reflux (12), dans lequel
la partie supérieure de la tour de distillation discontinue (1) est reliée au condenseur en haut de la tour (3), une sortie du condenseur en haut de la tour (3) est reliée au condenseur sous vide en haut de la tour (4), et une autre sortie du condenseur en haut de la tour (3) est reliée au dispositif de commande de reflux (12) ;
une sortie du dispositif de commande de reflux (12) est reliée à la tour de distillation discontinue (1), et une autre sortie du dispositif de commande de reflux (12) est reliée au réservoir de réception d'eau (9), au réservoir de réception de produit intermédiaire (10) et au réservoir de réception de produit (11) ;
la pompe de transfert de matière première et de produit intermédiaire (7) est reliée à la tour de distillation discontinue (1), au réservoir de réception de produit intermédiaire (10) et à une conduite d'alimentation en matière première (S1) ;
la pompe de rebouilleur (5) est reliée à la tour de distillation discontinue (1) afin de former un passage de circulation de désoxygénation, dans lequel la pompe de rebouilleur (5) a une entrée de gaz conçue pour introduire de l'azote, par le passage de circulation de désoxygénation, la pompe de rebouilleur pompe un liquide résiduaire de 1-méthyl-2-pyrrolidone hors de la tour de distillation discontinue et renvoie le liquide résiduaire de 1-méthyl-2-pyrrolidone vers un bouilleur de tour de la tour de distillation discontinue après le mélange de l'azote introduit par la pompe de rebouilleur (5) avec le liquide résiduaire de 1-méthyl-2-pyrrolidone ; et
la pompe de rebouilleur (5) est également reliée au rebouilleur d'évaporation à flux tombant de grattoir (2) afin de former un passage destiné à transférer le liquide résiduaire de 1-méthyl-2-pyrrolidone vers le rebouilleur d'évaporation à flux tombant de grattoir (2) pour le chauffage et l'évaporation, et le rebouilleur d'évaporation à flux tombant de grattoir (2) est relié à la tour de distillation discontinue (1), de sorte qu'une partie en phase gazeuse du liquide résiduaire de 1-méthyl-2-pyrrolidone entre dans une colonne de la tour de distillation discontinue (1), et qu'une partie en phase liquide du liquide résiduaire de 1-méthyl-2-pyrrolidone qui n'est pas vaporisée entre dans le bouilleur de tour de la tour de distillation discontinue, et la pompe de rebouilleur (5) est une pompe magnétique de mélange gaz-liquide.

2. Dispositif de distillation discontinue de 1-méthyl-2-pyrrolidone selon la revendication 1, comprenant en outre une pompe de transfert d'eau (6) reliée au réservoir de réception d'eau (9).

3. Dispositif de distillation discontinue de 1-méthyl-2-pyrrolidone selon la revendication 1, comprenant en outre une pompe de transfert de produit (8) reliée au réservoir de réception de produit (11).

4. Dispositif de distillation discontinue de 1-méthyl-2-pyrrolidone selon la revendication 1, dans lequel la tour de distillation discontinue (1) est remplie d'un matériau de remplissage à mailles BX500, et une hauteur du matériau de remplissage est comprise entre 3 et 6 m.

5. Procédé de distillation discontinue de 1-méthyl-2-pyrrolidone sur la base du dispositif de distillation discontinue de NMP selon l'une quelconque des revendications 1 à 4, comprenant une étape de prétraitement et une étape de distillation, dans lequel l'étape de prétraitement comprend les étapes suivantes consistant à :
transférer un liquide résiduaire de 1-méthyl-2-pyrrolidone vers la tour de distillation discontinue (1) à l'aide de la pompe de transfert de matière première et de produit intermédiaire (7) ;
introduire de l'azote dans la tour de distillation discontinue (1) afin de remplacer de l'air dans la tour de distillation discontinue (1) ;
démarrer un système de vide de la tour de distillation discontinue (1) afin de maintenir une pression de 8 à 9 kPa au niveau de la partie supérieure de la tour, et démarrer un système de chauffage à cheminée du bouilleur de tour afin d'effectuer un reflux total au niveau de la partie supérieure de la tour ;
effectuer un traitement de désoxygénation lorsqu'une température du liquide résiduaire de 1-méthyl-2-pyrrolidone dans le bouilleur de tour de la tour de distillation discontinue (1) atteint 0 à 6 °C en dessous d'une température de point de bulle, dans lequel une procédure du traitement de désoxygénation est la suivante : la pompe de rebouilleur (5) pompe le liquide résiduaire de 1-méthyl-2-pyrrolidone hors de la tour de distillation discontinue, et renvoie le liquide résiduaire de 1-méthyl-2-pyrrolidone vers le bouilleur de tour de la tour de distillation discontinue (1) après le mélange de l'azote avec le liquide résiduaire de 1-méthyl-2-pyrrolidone ;
après la fin du traitement de désoxygénation, fermer l'entrée de gaz de la pompe de rebouilleur (5) et un passage entre la pompe de rebouilleur (5) et la tour de distillation discontinue (1), et ouvrir un passage entre la pompe de rebouilleur (5) et le rebouilleur d'évaporation à flux tombant de grattoir (2), afin de chauffer et d'évaporer le liquide résiduaire de 1-méthyl-2-pyrrolidone ;
pendant la procédure de chauffage, le rebouilleur d'évaporation à flux tombant de grattoir (2) évapore le liquide résiduaire de 1-méthyl-2-pyrrolidone, une partie en phase gazeuse entre dans la colonne de la tour de distillation discontinue (1), et une partie en phase liquide qui n'est pas vaporisée entre dans le bouilleur de tour de la tour de distillation discontinue (1) ; et
lorsqu'une température du bouilleur de tour de la tour de distillation discontinue (1) atteint une température spécifiée et qu'une température de reflux de la tour de distillation discontinue (1) est stable, effectuer l'étape de distillation, dans lequel l'étape de distillation comprend les étapes suivantes consistant à :
ouvrir un passage entre le dispositif de commande de reflux (12) et le réservoir de réception d'eau (9), maintenir la pression au niveau de la partie supérieure de la tour de distillation discontinue (1) inchangée, et ajuster un rapport de reflux et une puissance de chauffage, afin d'effectuer l'extraction d'eau ;
fermer le passage entre le dispositif de commande de reflux (12) et le réservoir de réception d'eau (9), ouvrir un passage entre le dispositif de commande de reflux (12) et le réservoir de réception de produit intermédiaire (10), et ajuster le rapport de reflux, la puissance de chauffage et la pression au niveau de la partie supérieure de la tour de distillation discontinue (1), afin d'effectuer l'extraction de produit intermédiaire ; et
fermer le passage entre le dispositif de commande de reflux (12) et le réservoir de réception de produit intermédiaire (10), ouvrir un passage entre le dispositif de commande de reflux (12) et le réservoir de réception de produit (11), maintenir la pression au niveau de la partie supérieure de la tour de distillation discontinue (1) inchangée, et ajuster le rapport de reflux et la puissance de chauffage, afin d'effectuer l'extraction de produit 1-méthyl-2-pyrrolidone.

6. Procédé de distillation discontinue de 1-méthyl-2-pyrrolidone selon la revendication 5, dans lequel la procédure du traitement de désoxygénation est répétée au moins deux fois lors de l'étape de prétraitement.

7. Procédé de distillation discontinue de 1-méthyl-2-pyrrolidone selon la revendication 5, dans lequel, lors de l'extraction d'eau, la puissance de chauffage du système de chauffage à cheminée est à pleine charge, la pression au niveau de la partie supérieure de la tour de distillation discontinue (1) est de 8 à 9 kPa, et le rapport de reflux au niveau de la partie supérieure de la tour de distillation discontinue (1) est ajusté entre 0,1 et 0,3.

8. Procédé de distillation discontinue de 1-méthyl-2-pyrrolidone selon la revendication 5, dans lequel, lors de l'extraction de produit intermédiaire, la puissance de chauffage du système de chauffage à cheminée est de 30 % à 40 % de la pleine charge, la pression au niveau de la partie supérieure de la tour de distillation discontinue (1) est réduite de 8 à 9 kPa à 2 à 3 kPa, et le rapport de reflux au niveau de la partie supérieure de la tour de distillation discontinue (1) est ajusté entre 0,2 et 0,4.

9. Procédé de distillation discontinue de 1-méthyl-2-pyrrolidone selon la revendication 5, dans lequel, lors de l'extraction de produit de 1-méthyl-2-pyrrolidone, la puissance de chauffage du système de chauffage à cheminée est de 30 % à 40 % de la pleine charge, la pression au niveau de la partie supérieure de la tour de distillation discontinue (1) reste inchangée à 2 à 3 kPa, et le rapport de reflux au niveau de la partie supérieure de la tour de distillation discontinue (1) est ajusté entre 0,5 et 1,2.
